(19) Europäisches Patentamt / European Patent Office / Office européen des brevets

(11) **EP 2 258 727 A1**

(12) **EUROPEAN PATENT APPLICATION**

| | |
|---|---|
| (43) Date of publication:<br>08.12.2010 Bulletin 2010/49 | (51) Int Cl.:<br>*C07K 16/42* [(2006.01)]   *C07K 1/22* [(2006.01)]<br>*A61K 39/395* [(2006.01)] |
| (21) Application number: 09161247.3 | |
| (22) Date of filing: 27.05.2009 | |

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK TR**<br>Designated Extension States:<br>**AL BA RS** | (72) Inventors:<br>• **Concas, Daniela**<br>  **09131 Cagliari (IT)**<br>• **Metlas, Radmila**<br>  **09131 Cagliari (IT)**<br>• **Srdic, Tanja**<br>  **09131 Cagliari (IT)** |
| (71) Applicant: **Wezen Biopharmaceuticals S.r.l.**<br>**A Socio Unico**<br>**09131 Cagliari (IT)** | (74) Representative: **Banfi, Paolo**<br>**Bianchetti Bracco Minoja S.r.l.**<br>**Via Plinio, 63**<br>**20129 Milano (IT)** |

(54) **Immunoglobulin preparation for the treatment of autoimmune diseases and immune system disorders**

(57)    The invention provides an immunoglobulin preparation containing natural polyclonal IgG-reactive antibodies (Abs) isolated from normal human serum (NHS), for the treatment of autoimmune diseases and immune system disorders.

The IgG-reactive Abs potently neutralize disease-associated or pathogenic autoantibodies present in sera of patients suffering from autoimmune diseases, by binding to their antigenic determinants located either within or near (e.g. overlapping with) the antigen combining sites.

EP 2 258 727 A1

**Description**

The present invention relates to an immunoglobulin (Ig) preparation for therapeutic use in autoimmune diseases and immune system disorders. This Ig preparation contains polyclonal IgG-reactive antibodies (Abs), which have the features of connected Abs. The polyclonal IgG-reactive Abs in this preparation react with a common matrix used for IgG purification and display only slight rheumatoid factor (RF) activity. These IgG-reactive Abs potently neutralize disease-associated or pathogenic autoantibodies present in sera of patients suffering from autoimmune diseases, by binding to their antigenic determinants located either within or near (e.g. overlapping with) the antigen combining sites.

The idiotopes, possibly of germline configuration, which are instrumental for the predicted immunoregulatory role of IgG-reactive Abs might be predominantly encoded in the framework (FW) and/or CDR1 and CDR2 residues. These IgG-reactive Abs are isolated from pooled normal human serum (NHS) based on their reactivity with NHS IgG. The preparation is enriched in $IgG_2$ monomers and dimers.

Another aspect of the invention concerns the use of the Ig preparation according to the invention as a medicinal product. This medicinal product is particularly suitable for treating autoimmune diseases such as Systemic Lupus Erythemathosus (SLE), Hashimoto's Thyroiditis (HT) and Immune-Mediated Thrombocytopenic Purpura (ITP).

## BACKGROUND OF THE INVENTION

Autoimmune diseases are a poorly understood group of disorders which are usually considered to be the consequence of breakage of self tolerance, resulting in the appearance of autoreactive lymphocytes. Regulatory malfunctions of the immune system, although believed to be of a multigenetic predisposition (Davidson A and Diamond B, 2001), still need triggering events which are often unknown but which, in several diseases, may share common pathogenic pathways. This suggests the possibility of using common therapeutic strategies in different autoimmune diseases.

B-cell dominant and T-cell dominant responses have been described in autoimmune diseases. However, co-stimulatory help of T cells is required for the activation of autoreactive B cells (Murakami M and Honjo T, 1997).

In B cell-mediated diseases, pathogenic antibodies directed against self-antigens are frequently found in the serum of patients and are implicated in the destruction of the target tissues.

In murine models, autoimmune diseases can be induced by the transfer of autoantibodies or by immunization with anti-idiotypic autoantibodies (Shoenfeld Y, 1996).

Immune-mediated thrombocytopaenic purpura (ITP), Graves' disease, Hashimoto's thyroiditis (HT), autoimmune haemolytic anemia (AHA) and myasthenia gravis (MG) are examples of B-cell-mediated autoimmune diseases. Experimental autoimmune encephalomyelitis (EAE), type 1 diabetes (T1D), rheumatoid arthritis (RA) and multiple sclerosis (MS) are known to be T-cell dominant autoimmune diseases. Systemic lupus erythematosus (SLE) results from the emergence of autoreactive T and B cells, and is regarded as a complex disease with an aetiology that appears to be the interplay of genetic and environmental factors.

The current view is that a low level of autoantibodies is physiological. This indicates that autoreactive B and T cells specific for self-epitopes are components of normal lymphocytic repertoire (Bockenstedt LK et al., 1995) since they have been neither deleted nor irreversibly anergized (Cancro MP and Kearney JF, 2004). Thus, human serum contains "natural" (Boyden SV, 1966) or "innate" (Rodman TC, 1997) antibodies (Abs) that have not arisen as a result of direct immunization with or pathogenic exposure to, a foreign antigen (Ag) (Casali P and Schettino EW, 1996). These natural Abs are produced by the so-called B-1a (CD5+) subset cells, a predominant B cell population early in life. An important feature of these natural polyreactive autoantibodies is their ability to bind not only self antigens but also non-self antigens such as microbial molecules. Since autoreactivity is physiological and there is no fundamental difference between the structure of some self antigens (or autoantigens) and that of foreign antigens, it has been proposed that lymphocytes evolved not to distinguish self from foreign (Dighiero G and Rose NR, 1999), but rather to respond to antigens only in certain microenvironments (Silverstein AM and Rose NR, 2000), such as the marginal zone compartment (MZ) in which B cells participate in responses to T-cell-independent polysaccharide antigens and to encapsulated bacterial pathogens (Martin F, Oliver AM, Kearney JF., 2002; Kreutzmann S, et al., 2003), by producing primarily $IgG_2$. An over-representation of self-like antibodies in the natural antibody repertoire prevents an evoked immune reaction from binding to self epitopes. This supports the concept of a functional idiotypic network which regulates autoimmune responses (Melero J et al., 1997).

Consistent with this concept, it was observed that intravenous immunoglobulin (IVIg) contains a series of anti-idiotypic antibodies capable of inhibiting the in vitro activity of various autoantibodies (Rossi F et al., 1989). The observation that platelet counts increased after IVIg administration was the first evidence of an immunomodulatory effect of IVIg (Imbach P et al., 1981). Currently IVIg is an accepted treatment for several autoimmune diseases. While its beneficial effects are well documented, both the mechanisms leading to and the component(s) within the IVIg preparation responsible for its immunomodulatory effect are incompletely understood. IVIg is an extremely complex preparation which possesses various biological activities, due mostly to natural antibodies and to induced antibodies. It is remarkable that IVIg therapy is so widely used clinically despite the paucity of information about the mechanism of action of its biologically active component(s) on the immune system (Dwyer JM, 1992; Fuch S et al., 2007).

IVIg contains antibodies reactive with idiotypes of natural and of autoimmune disease-related autoantibodies, i.e. surface immunoglobulins (Igs) of B cells, as well as with conserved complementary idiotypes encoded by germline V genes, the so-called unconventional binding sites in the variable (V) region (Kohler H, 2000). This evidence prompted us to postulate that IgG-reactive Abs, and in particular.

IgG-reactive $IgG_2$, may also be involved in such interactions.

It is still unclear whether loss of B cell tolerance in patients with autoimmune diseases is due to underlying defects in the molecular mechanisms involved in the arrangement of antibody genes or to deficiencies in the subsequent selective influences that shape the antibody repertoire. Analysis of immunoglobulin (Ig) variable (V) gene usage has identified recombinatorial bias such that only seven VH genes, mostly of the VH3 and VH4 families, make up almost 60% of the productive peripheral B cell repertoire. Since no obvious differences between the B cell receptor gene segments found in autoimmune diseases and in normal controls have been shown so far, evidence suggests that $V_H$-gene representation in autoantibodies reflects the normal human B cell repertoire (Stewart AK at al., 1992; Kohsaka H et al., 1996.

Thus, the systematic analysis of human autoantibody sequences suggests that the $V_H$ gene repertoire is genetically controlled and mostly unaltered by chronic autoimmune stimulation. An alternative proposal is that somatic hypermutation might be responsible for the production of autoimmune disease-associated IgG, even if a high mutation frequency is not always necessary for the development of high-affinity Abs (Foreman AL et al., 2007).

The suppression of autoimmune disease involves multiple checkpoints. Natural T regulatory cells (nTregs) play an important role. We now postulate that IgG-reactive $IgG_2$ dimers contain regions or epitopes that are stimulatory to nTregs.

### Systemic lupus erythematosus (SLE)

SLE is a systemic autoimmune disease resulting from immune-mediated damage to multiple organs. Impaired immune regulation permits self-reactive T cell dependent B cell activation, which results in the production of autoantibodies. Serum anti-double-stranded (ds) DNA antibodies, which bear the common idiotype 16/6, are generally regarded as markers of SLE, and are responsible for many clinical manifestations of SLE, including glomerulonephritis and arthritis (Hahn BH, 1998). The utility of anti-dsDNA antibodies in predicting disease relapses and the efficacy of therapeutic agents has been extensively assessed (Swaak T and Smeenk R, 1985; Munoz LE, Gaipl US, Herrmann M, 2008). DNA, to which most of the autoantibodies are directed in SLE, is no longer regarded as the antigen initiating the disease (Blank M and Shoenfeld Y, 2008), mainly because immunization of mice with DNA could not induce SLE (Madaio, MP et al., 1984). A large proportion (about 40%) of patients with SLE have increased levels of serum Ig encoded by the VH4-34 heavy chain gene, which binds to a range of antigens via two distinct sites, namely via the complementary-determining region 3 (CDR3) or via the framework region 1 FWR1 (Li Y et al., 1996). Anti-DNA antibodies encoded by the VH4-34 gene segment have positively charged amino acids in the CDR3 region which favour interaction with DNA. However, these positively charged amino acids appear to suppress the ability of the FW region to interact with the poly-N-acetyl-lactosamine determinants of the I/i blood group antigens (Spellerberg MB et al., 1995).

Individuals with systemic autoantibody-mediated diseases, such as SLE, seem to have polyclonal T and B cell activation. Yet, autoantibody production is restricted to certain autoantigens. The mechanisms underlying this phenomenon remain unclear but indicate that multiple mechanisms of polyclonal T cell activation may operate, at least in murine SLE (Sing RR et al., 1998). It has been shown that young SLE prone mice develop spontaneous T cell activation to several peptides derived from syngeneic autoantibody heavy chain variable (VH) regions. Identical sequences of such determinants were not found in murine proteins other than Ig (Singh RR et al., 1998). These determinants occur infrequently in non-autoantibody Ig using the same VH gene family, but quite commonly in SLE-related autoantibodies such as Abs to DNA, cardiolipin, or erythrocytes (Singh RR, 1995; Ebling FM et al., 1993). Similar spontaneous T cell responses are not observed in normal mouse strains (Singh RR, 1995). Another line of evidence does not support the role of polyclonal B cell activation in SLE development but suggests the involvement of a superantigen specific for B cells bearing VH4 immunoglobulin (Demaison C et al., 1996).

Non-autoimmune mice can be induced to develop antibodies to DNA and mild nephritis by in vivo stimulation of the Th cells which are capable of promoting autoantibody production. Complete recovery from such an episode of autoimmunity, despite persistent exposure to autoreactive Th cells, correlates with the appearance of certain $CD8^+$ T, $CD4^+$ $CD25^+$ T (Tregs) and natural killer T (NKT) cells which are capable of suppressing autoantibody production (Singh RR, 2005). However, immunization with the human anti-DNA 16-6 idiotype results in an experimental SLE in naive mice (Mendlovic S et al., 1988).

These data illustrate the complexity of this autoimmune disease which is further illustrated by the observation that the anti-idiotype Abs which arise naturally during the development of SLE react both with autoantibody idiotopes and with dsDNA (Eivazova ER et al., 2000). As these anti-idiotype Abs bind also to dsDNA, the idiotopes on anti-DNA Abs in SLE may act as natural mimotopes able to induce anti-dsDNA antibodies. Thus, it is suggested that anti-DNA antibodies may arise inadvertently as a response to antibodies generated against microorganisms (Wun HL et al., 2001).

Because many pathogenic autoantibodies bind DNA, there have been attempts to tolerize or turn off DNA-specific B cells. A tolerogenic peptide named hCDR1 was found to down-regulate cytokines, and to up-regulate immunosupressive molecules and Tregs, in peripheral blood mononuclear cells of SLE patients (Sthoeger ZM et al., 2009).

Despite encouraging case reports and uncontrolled studies, the clinical value of IVIg in the treatment of SLE patients is not well established. The reported beneficial effects of IVIg are usually of limited duration. Clinical response can only be maintained by repeated monthly IVIg infusions. At present, IVIg is used in severe SLE cases refractory to other therapies, or as a steroid-sparing agent. The efficacy of IVIg in maintaining SLE improvement remains to be demonstrated (Toubi E, Kessel A and Shoenfeld Y, 2005). Renal failure was observed to occur in association with IVIg treatment in SLE (Ahsan N, 1998).

The therapeutic dose of IVIg in SLE is set at 2g/kg divided into five daily doses of 400mg/kg each in order to prevent the risk of adverse events. Concentrated specific natural polyclonal anti-dsDNA anti-Id Abs separated from commercial IVIg were tested in NZB/WF1 mice. Treatment with such specific Abs was shown to be more efficacious in suppressing the humoral reaction and clinical signs of SLE than was IVIg (Shoenfeld Y et al., 2002).

In the context of the present invention, we propose treating SLE patients with the IgG-reactive Abs preparation we describe below, in order to neutralize autoantibodies and to activate Tregs. The latter effect is predicted to have an impact on prolonging the reduction in autoantibody levels.

### *Hashimoto's thyroiditis*

Hashimoto's thyroiditis (HT), also known as chronic autoimmune thyroiditis, is an autoimmune thyroid disease of unknown aetiology with a hereditary link. In HT, antibodies react against proteins in the thyroid gland, causing gradual destruction of the gland itself, and making the gland unable to produce the thyroid hormones the body needs. Thus, HT is associated with the presence of organ specific autoantibodies. Three anti-thyroid autoantibodies have been identified: anti-thyroglobulin (Tg) autoantibodies, anti-thyroid peroxidase (TPO) autoantibodies (also known as anti-thyroid microsomal antibodies) and anti-thyroid stimulating hormone (TSH) receptor autoantibodies. In population surveys, anti-TPO antibodies and anti-Tg antibodies are the more common autoantibodies which can be demonstrated in patients' sera. Anti-TPO and anti-Tg antibodies from patients with HT are of IgG class and have high affinities for their respective autoantigens (McLachlan SM and Rapoport B, 2004). Heavy (H) chain variable (V) domains of anti-TPO antibodies in HT are characterized more frequently by the VH3 gene. In contrast to the numerous somatic hypermutations in the TPO-specific antibody heavy chains, there is only limited amino acid replacement in most of the TPO-specific antibody light chains. Thus, conserved somatic mutations are the hallmark of the anti-TPO antibody repertoire (Chardès T et al., 2002).

Thyroglobulin is a 660 kDa dimeric protein. At a molecular level, anti-Tg autoantibodies show considerable heterogeneity even if directed to the same or a closely related epitope. In apparently healthy blood donors, anti-Tg autoantibodies can be found which are directed against two major conformational epitopes on the Tg molecule, which differ from the epitopes targeted by the anti-Tg autoantibodies found in patients with autoimmune thyroiditis (Prentice L et al., 1995).

In autoimmune thyroid disease, thyroid peroxidase is one of the main autoantigenic targets. Membrane TPO is a two subunit molecule. This highly complex molecule comprises three distinct domains involved in antibody binding (Bresson D et al, 2005). Anti-TPO autoantibodies recognize conformation-dependent epitopes.

Some studies suggested that anti-thyroid antibody level elevation is proportional to the degree of disease activity, since levels have been shown to decrease after treatment with steroids (Canton A et al., 2000).

IVIg has been shown to compete with monoclonal or polyclonal anti-idiotypic reagents for binding to anti-Tg autoantibodies by recognizing an immunodominant cross-reactive alpha idiotype which is specifically expressed by autoantibodies from patients with HT. The expression of this disease-associated idiotype correlates with the recognition of restricted epitopic determinants on human Tg (Dietrich G and Kazatchkine MD, 1990). Cases of Hashimoto's thyroiditis, and in particular of Hashimoto's encephalopathy have been treated with periodic IVIg based on its probable clinical benefit.

We propose treating Hashimoto's thyroiditis with IgG-reactive Abs primarily to reduce the level of Tg and TPO reactive autoantibodies, as well as to suppress the activity of B cells producing such autoantibodies.

### *Immune-mediated Thrombocytopenic Purpura (ITP)*

ITP is a common immuno-hematological bleeding disorder caused by clonally restricted platelet-reactive autoantibodies with specificity for platelet glycoproteins (Beardsley DS, 2006). ITP affects adults and children. In ITP, anti-platelet autoantibodies belong mainly to the IgG class, although IgM anti-platelet autoantibodies have also been reported (Stahl D et al., 2005). In ITP, clearance of antibody-coated platelets by tissue macrophages is accelerated. In some cases, autoantibodies impair platelet production (Chang M et al., 2003). ITP is associated with the presence of autoantibodies directed against various platelet membrane receptors, including platelet glycoproteins such as glycoprotein IIb/IIIa (GP IIb/IIIa) or GP Ib/IX complexes. Antibodies against platelet glycoproteins (anti-GP) are found in the majority of patients with ITP, as well as in thrombocytopenia associated with systemic lupus erythematosus (SLE). Some ITP patients have anti-phospholipid (anti-PL) antibodies. The presence of anti-GP IIb/IIIa antibodies correlates with the severity of thrombocytopenia (Lipp E et al., 1998). Approximately 75% of platelet autoantigens are located on either the platelet glycoprotein (GP) IIb/IIIa or Ib/IX complex. Many platelet-associated and plasma autoantibodies from ITP patients are light chain-restricted, which suggests a clonal origin (McMillan R et al., 2000).

However, other platelet antigens also appear to be targeted (Hou M, 1995). In many cases, the antibody specificity cannot be determined or even detected. Platelet clearance is mediated by receptor-bearing (FcγR) macrophages in the reticuloendothelial system (RES). Human macrophages express several Fc receptors that bind IgG specifically (Crow AR and Lazarus AH, 2003). FcγRI, the high affinity receptor, does not appear to play a role in ITP. Engagement of FcγRIIA on the surface of human macrophages by anti-GPIIb/IIIa-coated platelets triggers intracellular signaling that leads to engulfment of the opsonized platelets. FcγRIIB shares considerable homology with FcγRIIA (both have subclass specificity for $IgG_1$, $IgG_2$ and $IgG_3$), but the former promotes inhibitory signaling upon binding of the Fc portion of an immune complex.

Treatment of ITP with IVIg is well established. However there is no consensus on the mechanism of action of IVIg in ITP, which may involve "competitive" RES blockade or mainly inhibitory signaling through the FcγRIIB pathway (Bussel JB, 2000; Crow AR et al., 2003). According to Siragam V et al. (2005), the beneficial effect of IVIg in ITP treatment might also be achieved by means of antibodies directed against soluble antigens like albumin or transferrin. The mechanism of the therapeutic effect of IVIg in the clinical setting of human ITP is probably more complex and may include contributions from anti-idiotypic antibodies, FcR blockade, and alteration of autoantibody production and clearance.

Numerous studies have been performed to characterize the pathogenic autoantibodies responsible for platelet destruction. While normal B cells do not produce anti-platelet antibodies in vivo, it has been demonstrated that in vitro such cells have the potential to produce anti-GPIIb and anti-PL antibodies which have antigen specificities similar to those of disease-associated antibodies. Anti-GP and anti-PL antibodies are encoded by un-mutated VH4 germline genes with N-nucleotide additions in the CDR3 regions. Therefore it is possible that in ITP, naturally occurring antibody producing B cells are recruited to develop pathogenic autoantibodies (Denomme GA et al., 1994). However, Roark JH et al. (2002), by phage display methods, found platelet-specific autoantibodies whose VH gene usage was restricted to the VH3 (VH3-30) gene.

We propose treating ITP with IgG-reactive Abs which target and neutralize anti-platelet antibodies. In addition, such IgG-reactive Abs, may cross-link the inhibitory FcγRIIb receptor expressed on B cells and inhibit production of anti-platelet antibodies.

## DISCLOSURE OF THE INVENTION

The present invention provides an immunoglobulin preparation containing IgG-reactive Abs isolated from pooled normal human serum

(NHS), for use in the treatment of autoimmune diseases and immune system disorders.

The invention provides natural polyclonal connected IgG-reactive immunoglobulins (Igs) present in NHS which are able to neutralize pathogenic autoantibodies present in patients' sera and are important for the suppression of autoantibody production and the activation of nTregs.

These IgG-reactive Abs are isolated from pooled NHS by affinity chromatography due to their reactivity with NHS IgG. According to a first embodiment, the isolation process comprises the following steps:

(a) passing NHS through a chromatographic matrix that specifically binds human IgG, preferably GammaBind G Sepharose matrix, and eluting the matrix-bound IgG fraction to obtain affinity purified IgG;
(b) covalent binding of one aliquot of the affinity purified IgG obtained in (a) to CNBr-activated Sepharose 4B, or an equivalent matrix, to obtain IgG-Sepharose;
(c) contacting a second aliquot of the affinity purified IgG obtained in (a) with the IgG-Sepharose obtained in (b) and eluting IgG-Sepharose bound Igs to prepare IgG-reactive IgG.

In another embodiment, the IgG-reactive Abs are isolated as follows:

(a') passing NHS through a GammaBind G Sepharose matrix and recovering the matrix-bound IgG fraction and the matrix-unbound fraction or "GammaBind G flow-through" (GBF) fraction;
(b') eluting the matrix-bound IgG fraction, obtained as in (a'), to obtain affinity purified IgG;
(c') covalent binding of the affinity-purified IgG obtained in (b') to CNBr-activated Sepharose 4B, to prepare IgG-Sepharose;

(d') contacting the GBF fraction obtained in (a') with IgG-Sepharose and eluting the IgG-Sepharose bound Igs to obtain IgG-reactive GBF Abs.

In a preferred embodiment, the IgG-reactive IgG (step (c)) and the IgG-reactive GBF Abs (step (d')) are combined into a single preparation.

The IgG-reactive Abs preparations consisting of IgG-reactive IgG and/or IgG-reactive GBF Abs are able to react with molecules of superimmunoglobulin family members and define a biologically active, immunoregulatory, component of total NHS Igs.

The IgG-reactive Abs preparations consisting of IgG-reactive IgG and/or IgG-reactive GBF Abs contain only traces of RF. Healthy individuals express IgM RF of low affinity on peripheral B cells but fail to express the higher affinity "pathologic" RFs which are associated with diseases such as rheumatoid arthritis (RA). Rheumatoid factor-associated light-chain idiotypes are rare in serum IgG (Carson DA et al., 1987).

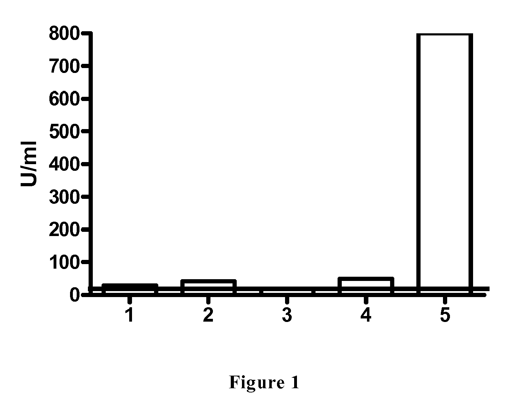

An assay specific for all RF isotypes (IgM, IgG and IgA) using the Fc portion immobilised on an ELISA plate showed a very low level of RF activity in IgG-reactive IgG, in IgG-reactive GBF Abs (which contain IgM and IgA) and in total un-fractionated NHS IgG at very high concentration (Fig. 1).

IgG-reactive IgG and IgG-reactive GBF Abs each inhibit, at low concentration,

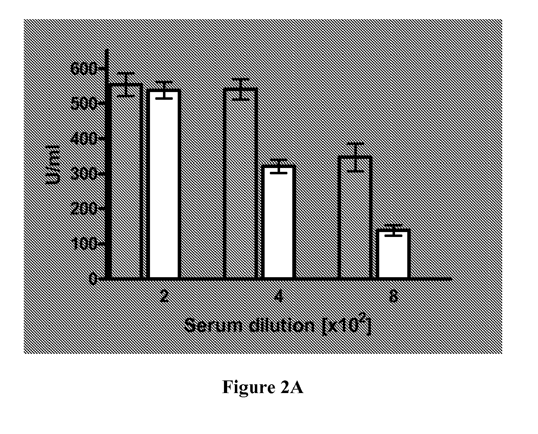

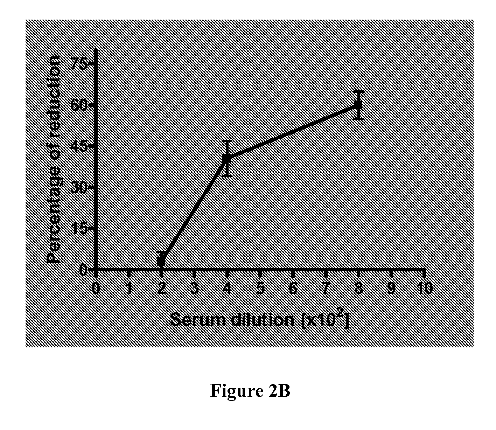

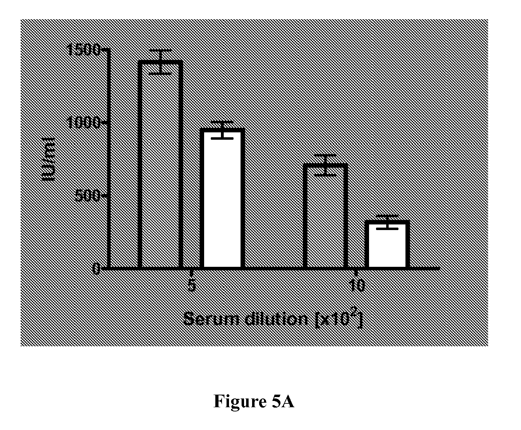

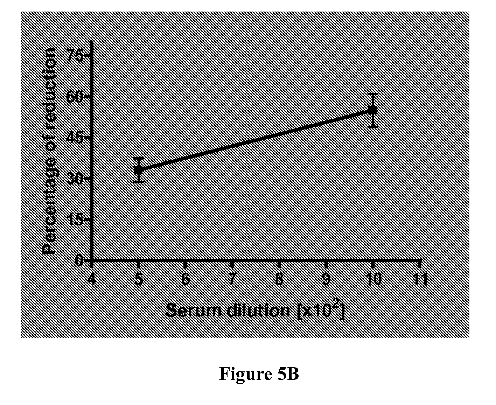

(a) binding of anti-dsDNA autoantibodies from SLE patients' sera to dsDNA immobilized on plates (Fig. 2-4);
(b) binding of anti-thyroid peroxidase (TPO) autoantibodies, and of anti-thyroglobulin (Tg) autoantibodies, from HT sera to TPO or Tg immobilized on plates (Fig. 5-10).

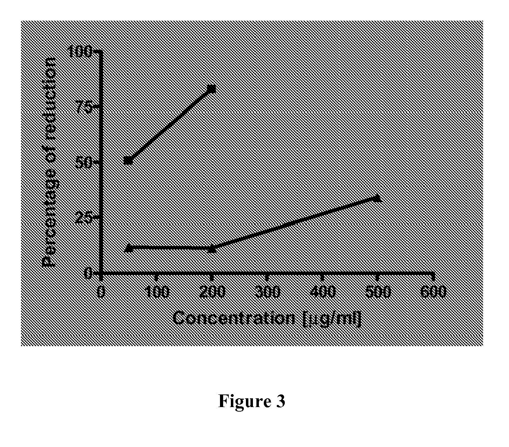

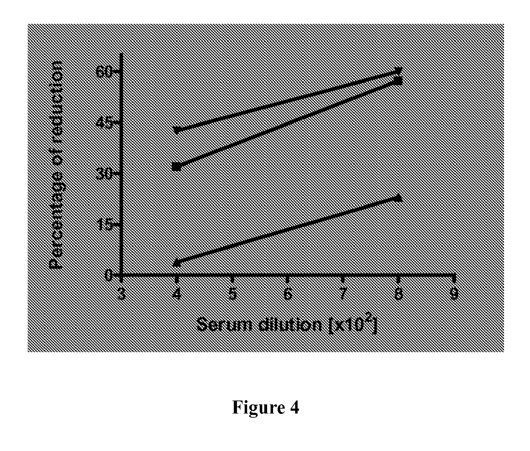

This neutralization capacity is dose-dependent (Fig. 3, 4, 6, 9) and is significantly higher than that displayed by an equivalent concentration of the total un-fractionated NHS IgG from which IgG-reactive antibodies are separated. Combining IgG-reactive IgG with IgG-reactive GBF Abs, preferably in a proportion equal to their respective serum amounts, generates an increase in the autoantibody neutralization capacity of the preparation (Fig. 4). Furthermore, combining IgG-reactive IgG with IgG-reactive GBF Abs may increase the immunoregulatory effect of the preparation.

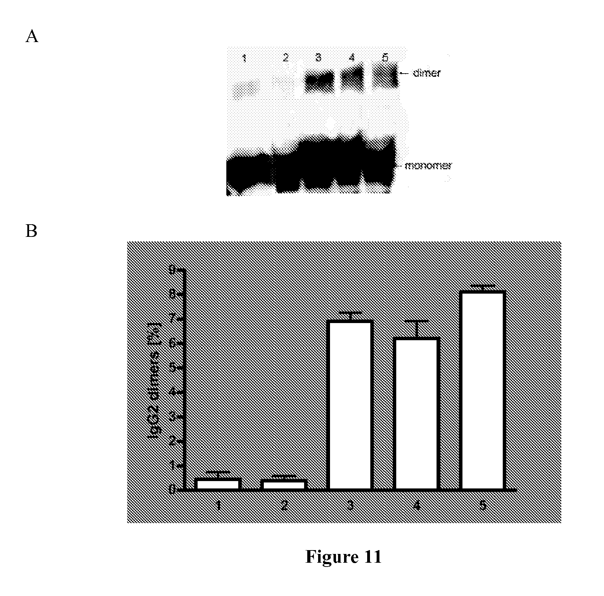

A very important characteristic of the IgG-reactive IgG and of IgG-reactive GBF Abs is the presence of an increased concentration of the $IgG_2$ subclass compared to that in NHS. This is independent of the source from which the IgG-reactive Abs are separated, i.e. from total NHS IgG or from the GBF fraction. Furthermore, a significant proportion of the $IgG_2$ contained in the preparation is in the form of covalent dimers (Fig. 11A and B).

Total NHS IgG and IgG-reactive Abs were compared for their content of $IgG_2$ dimers by Western blotting analysis using conditions that allow detection both of $IgG_2$ monomers and of $IgG_2$ dimers. A higher $IgG_2$ content was demonstrated both in IgG-reactive IgG and in the IgG-reactive GBF Abs, when compared to total un-fractionated NHS IgG. Densitometric measurement of the Western blot bands was performed and presented as the $IgG_2$ dimers percentage of the sum of integrated density (ID) values of $IgG_2$ monomers and dimers. The results revealed that $IgG_2$ dimers in total NHS IgG account for less than 1% compared to about 7% in IgG-reactive antibodies (Fig. 11B).

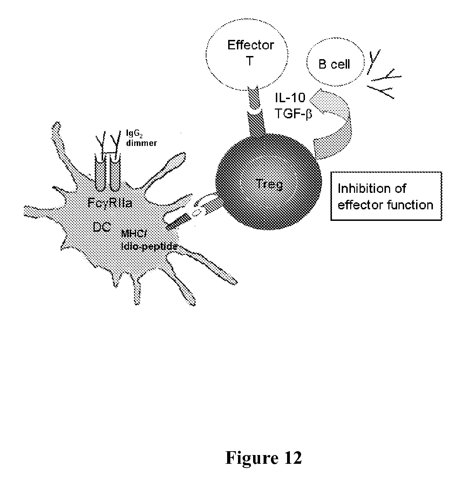

Thus, an important characteristic of IgG-reactive Abs is the presence of innate-like $IgG_2$ antibodies in the form of covalent dimers. The $IgG_2$ dimers present in IgG-reactive Abs may have an immunoregulatory function. IgG-reactive Abs, in addition to an immediate neutralization effect on disease-associated auto-Abs, and consequently on B cells producing such auto-Abs, may have a long lasting immunoregulatory activity. According to our model, IgG-reactive Abs have a fundamental role in the modulation of the expression of the autoimmune repertoire (Figure 12). Their long-lasting immunoregulatory effect might be achieved through the activation of naturally occurring $CD25^+CD4^+$ T regulatory and/or $CD8^+$ inhibitory T (Ti) cells (nTregs). $IgG_2$ as a homotypic aggregate may easily interact with activating FcγRIIa expressed by dendritic cells (DC) (Boruchov AM et al., 2005; Liu Y et al., 2006). $IgG_2$ molecule undergoes endocytosis, processing, and presentation of appropriate fragments in the context of Major Histocompatibility Complex (MHC) molecules. Processed fragments (idiotypes) may mimic self and/or foreign epitopes. These structures, possibly of foetal origin and germ line-encoded, might be located in the framework regions (FW) and/or in the complementarity-determining regions (CDR1 or CDR2). Ag-presenting DC may engage relevant nTregs in a cognate interaction which subsequently may down-regulate effector T cell via regulatory cytokines or cell to cell contact (Fig. 12). Effector B cells might be targets as well.

If the emergence of pathological autoimmunity is the consequence of a defect in the network of regulatory mechanisms operating in healthy individuals, then according to our model, rare mutations occurring in the FW and/or CDR1, CDR2 regions may be responsible for autoimmune disorders. In this context, data supporting the role of Ig regions out of the conventional Ag binding site were obtained using an artificial peptide (hCDR1) based on sequences common to several anti-dsDNA antibodies. Induction of regulatory T cells by the tolerogenic peptide hCDR1 was reported to be able to block the production of anti-dsDNA antibodies (Sharabi A and Mozes E, 2008; Sthoeger Z M et al., 2009).

Administration of IVIg has been shown to benefit patients in an ever-increasing number of autoimmune diseases (Kazatchkine MD, Kaveri SV, 2001). At present, ITP, Kawasaki disease, and Ouillain-Barré syndrome (GBS) are considered by the regulatory authorities as established indications for IVIg. Clinical benefit has been shown for numerous autoimmune neuromuscular diseases such as multifocal motor neuropathy (MMN), chronic inflammatory demyelinating polyradiculoneuropathy (CIDP), and myasthenia gravis (MG) (Dalakas MC, 2004; EFNS Guidelines). Other indications for IVIg currently under clinical testing include Alzheimer's disease (Solomon B, 2007).

The mode of action of IVIg involves several mechanisms that act in synergy which include non-specific blocking of Fcγ receptors expressed on cells of the reticulo-endothelial system; neutralization of circulating pathogenic autoantibodies; interaction with the anti-idiotypic network, re-establishment of immune regulation; modulation of the activation and differentiation of T cells, B cells and dendritic cells (DC) (Mouthon L. et al, 1996; Seite JF et al., 2008). IVIg contains a large repertoire of natural and induced antibodies. This raises the issue as to which component(s) are active in the treatment of autoimmune diseases. Evidence is accumulating that the therapeutic efficacy of IVIg in autoimmune disease depends on a minute fraction of the total Ig administered. This is the reason why "high dose" IVIg (1-2g/kg) is required for the therapeutic management of autoimmune conditions.

The preparation described in the invention has several advantages compared to currently available commercial IVIg. Administration of IgG-reactive Abs with neutralizing activity against pathogenic autoantibodies, and with immunoregulatory, i.e. nTregs or CD8$^+$ activation, properties, allows:

- lower Ig dose to be used to obtain a therapeutic effect. Given that the novel Ig preparation described in the invention consists mostly of Ig endowed with neutralizing activity against pathogenic autoantibodies and with an immunoregulatory function, the effective Ig dose may be reduced by a factor of 100, or even more, compared to IVIg;
- since the product is enriched in IgG$_2$ with immunoregulatory function, its effectiveness is superior to that of IVIg;
- since the preparation combines neutralizing Ig of short-term effect with immunoregulatory Ig of long lasting effect, its long-term therapeutic effect could be longer than that observed with IVIg;
- simplified administration compared to IVIg. The administration of very low doses makes possible subcutaneous (SC) or intramuscular (IM) injection and thus, outpatient treatment instead of intravenous (IV) infusion in a hospital environment;
- improved tolerance compared to IVIg. This advantage is particularly significant in the elderly, in very young recipients, in patients with cardiac impairment, in patients with renal dysfunction, and in patients at increased risk of a thromboembolic event. With the need for large doses of IVIg in autoimmune diseases, volume load becomes a safety issue. With lower doses, the tolerance of this novel product is improved compared to IVIg. In addition, slow infusion over several hours or days is avoided;
- the possibility of self or home treatment will improve patient's compliance and quality of life.

## RELATED PATENT DOCUMENTS

Nur I and Shoenfeld Y (WO 03/099868) disclose the use of immunoglobulins purified from IVIg as a tool for the selection of synthetic molecules which mimic autoimmune self-antibodies and as a source for the isolation of specific antibodies for therapeutic use in autoimmune disease.

Bourel D et al. (US 6,932,969) disclose a method for preparing Ig fractions from human polyvalent intravenous immunoglobulins (IVIg) which are likely to be responsible for the immunomodulatory effect observed during the treatment of certain autoimmune diseases.

Unlike the cited prior documents, the present invention provides the total connected antibodies present in NHS, which are isolated by affinity purification based on their reactivity with NHS IgG. The IgG-reactive Abs described in our invention combine neutralization of autoimmune disease-associated autoantibodies with immunoregulatory functions.

## DESCRIPTION OF THE FIGURES

### Figure 1. Binding of IgG-reactive antibodies and of total NHS IgG to the Fc portion

Binding to the Fc portion of IgG-reactive IgG and of IgG-reactive GBF antibodies was tested at a concentration of 50 g/ml and of 48 μg/ml (expressed on all Ig isotypes present), respectively. Binding of total NHS IgG to the Fc portion was assayed at concentrations of 50 μg/ml and of 500 μg/ml.

1- IgG-reactive IgG
2- IgG-reactive GBF Abs
3- Total NHS IgG (50μg/ml)
4- Total NHS IgG (500μg/ml)
5- Rheumatoid arthritis (RA) serum (100 fold dilution)

**Figure 2A. Binding of anti-dsDNA antibodies from a representative SLE patient's serum to immobilized dsDNA before and after co-incubation with IgG-reactive IgG;**

Binding was measured at three different serum dilutions before [-▥-] and after

[-□-] co-incubation with IgG-reactive IgG at a concentration of 50 µg/ml.

**Figure 2B.** Percentage reduction of binding of anti-dsDNA antibodies from a representative SLE patient's serum to immobilized dsDNA after co-incubation with IgG-reactive IgG.

The percentage reduction obtained represents the mean of two independent determinations. The SLE patient's serum shown is a representative serum of 4 SLE sera from 4 different patients with similar anti-dsDNA antibody levels.

**Figure 3. Dose-dependent reduction of anti-dsDNA antibodies binding to immobilized dsDNA after co-incubation with IgG-reactive IgG (-■-) and with total (un-fractionated) NHS IgG (-▲-).**

A representative SLE patient's serum with an anti-dsDNA antibody level of 580 U/ml was co-incubated with two different concentrations (50 µg/ml and 200 µg/ml) of IgG-reactive IgG and with three different concentrations (50 µg/ml, 200 µg/ml and 500 µg/ml) of total (un-fractionated) NHS IgG.

**Figure 4. Percentage reduction of binding of anti-dsDNA antibodies from a representative patient's serum to immobilized dsDNA after co-incubation with IgG-reactive IgG, with IgG-reactive GBF antibodies and with combined IgG-reactive IgG and IgG-reactive GBF antibodies.**

A representative SLE patient's serum with anti-dsDNA antibody levels of 450 U/ml (400 fold dilution) or of 302 U/ml (800 fold dilution) was co-incubated with IgG-reactive IgG at a concentration of 50 µg/ml (-■-) and with IgG-reactive GBF antibodies at a concentration of 4 vg/ml (-▲-). Co-incubation of the SLE patient's serum with a preparation consisting of combined IgG reactive IgG and IgG-reactive GBF antibodies at a concentration of 54 µg/ml (-▼-).

**Figure 5A. Binding of anti-TPO antibodies from a representative HT patient's serum to immobilized TPO before and after co-incubation with IgG-reactive IgG.**

Binding was measured at two different dilutions before [-▥-] and after [-□-] co-incubation with IgG-reactive IgG at a concentration of 50 µg/ml.

**Figure 5B. Percentage reduction of binding to of anti-TPO antibodies from a representative HT patient's serum to immobilized TPO after co-incubation with IgG-reactive IgG.**

The percentage reduction represents the mean of two independent determinations. The HT patient's serum shown is a representative serum of 3 HT sera from 3 separate patients with similar anti-TPO antibody levels.

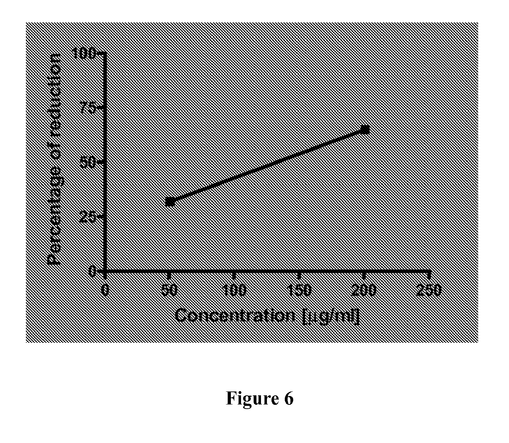

**Figure 6. Percentage reduction of binding of anti-TPO antibodies from a representative HT patient's serum to immobilized TPO after co-incubation with IgG-reactive IgG.**

A representative HT patient's serum containing anti-TPO antibodies at a level of 997 IU/ml was co-incubated with two different concentrations (50 µg/ml and 200 µg/ml) of IgG-reactive IgG.

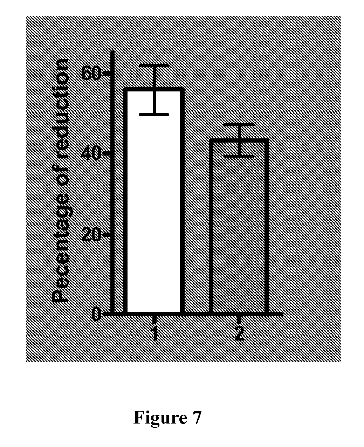

**Figure 7. Percentage reduction of binding of anti-TPO antibodies from a representative HT patient's serum to immobilized TPO after co-incubation with IgG-reactive IgG and with IgG-reactive GBF antibodies.**

A representative HT patient's serum with an anti-TPO antibody level of 922 IU/ml (1000 fold dilution) was co-incubated with IgG-reactive IgG at a concentration of 50 µg/ml (-□-) and with IgG-reactive GBF antibodies at a concentration of 8 µg/ml (-▥-). The percentage reduction obtained represents the mean of two independent determinations. The HT patient's serum shown is a representative serum of 3 HT sera from 3 different patients with similar anti-TPO antibody levels.

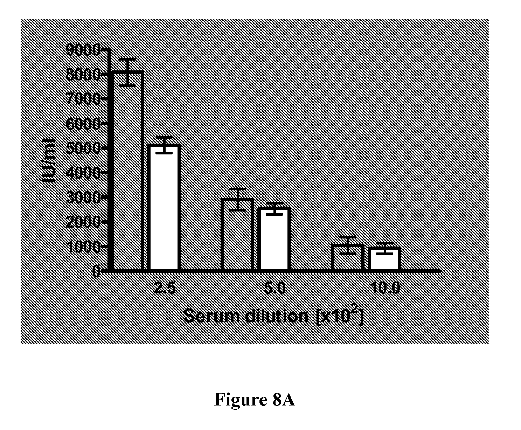

**Figure 8A. Binding of anti-Tg antibodies from a representative HT patient's serum to immobilized Tg before and after co-incubation withIgG-reactive IgG.**

Binding was measured at three different serum dilutions before [-▥-] and

**[0068]** after [-□-] co-incubation with 50 µg/ml of IgG-reactive IgG.

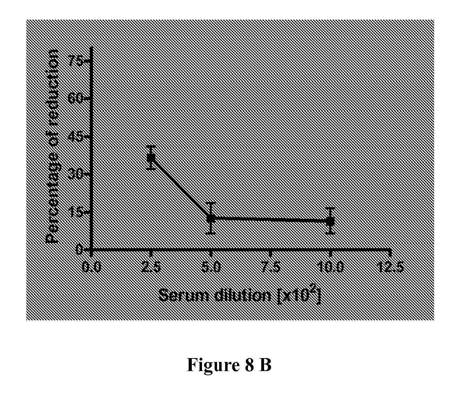

**Figure 8B. Percentage reduction of binding of anti-Tg antibodies from a representative HT patient's serum to immobilized Tg after co-incubation with IgG-reactive IgG.**

The percentage reduction obtained represents the mean of independent determinations. The HT patient's serum shown is a representative serum of 3 HT sera from 3 separate patients with similar anti-Tg antibody levels.

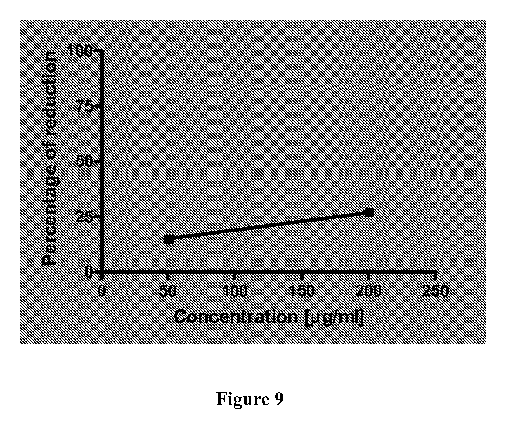

**Figure 9. Dose-dependent reduction of binding of anti-Tg antibodies from a representative HT patient's serum to immobilized Tg after co-incubation with IgG-reactive IgG.**

A representative HT patient's serum containing anti-Tg antibodies at a level of 2025 IU (100 fold dilution) was co-incubated with two different concentrations (50 $\mu$g/ml and 200 $\mu$g/ml) of IgG-reactive IgG.

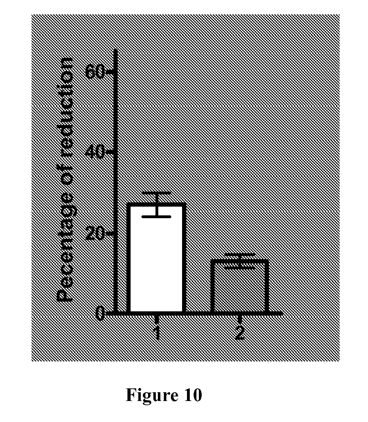

**Figure 10. Percentage reduction of binding of anti-Tg antibodies from a representative HT patient's serum to immobilized Tg after co-incubation with IgG-reactive IgG and with IgG-reactive GBF antibodies.**

A representative HT patient's serum with an anti-Tg antibody level of 8080 IU/ml (250 fold dilution) was co-incubated with IgG-reactive IgG at a concentration of 50 $\mu$g/ml (-□-) and with IgG-reactive GBF antibodies at a concentration of 6 $\mu$g/ml (-▥-). The percentage reduction obtained represents the mean of two independent determinations. The HT patient's serum is a representative serum of 3 HT sera from 3 different patients with similar anti-Tg antibody levels.

**Figure 11. Densitometric quantitation of the Western blot data.**

A: Western blotting analysis was performed by a procedure allowing detection of $IgG_2$ monomers and dimers.
Lane 1 - affinity purified IgG (10 $\mu$g/ well); Lanes 2 - affinity purified IgG (4 $\mu$g/well); Lane 3 - IgG-reactive IgG (4 $\mu$g/well); Lane 4 - IgG-reactive IgG (2 $\mu$g/well); Lane 5 - IgG-reactive GBF Abs (2 $\mu$g/ well). Data of a representative blot is shown.
B: Densitometric quantitation of the Western blot bands is presented as the percentage of the $IgG_2$ dimers of the sum of integrated density (ID) values of $IgG_2$ monomers and dimers. Results are presented as a mean $\pm$ SD from three independent experiments. Quantification of the blots was performed using a GS-710 Calibrated imaging densitometer (Bio-Rad) and the Image Quant software program.

**Figure 12. Postulated model of cognate interactions in the Tregs stimulation.**

In antigen (idiotype)-specific stimulation of anergic Tregs the first step is interaction between $IgG_2$ dimers and Fc$\gamma$RIIa receptor expressed on the surface of DC. After internalization and processing DC may effectively present MHC class II-associated peptide derived from variable region of $IgG_2$ i.e. idio-peptide (an Ag surrogate) possibly derived from framework region and/or CDR1 and CDR2. In the second step MHC-idio-peptide is recognized by TCR of the Tregs. Finally, suppression of Ag-specific effector cells by Tregs might be cell contact dependent or cytokine dependent.

**MATERIALS AND METHODS**

**Patients' sera**

Sera from patients with a confirmed diagnosis of Systemic Lupus Erythematosus (SLE) or Hashimoto's thyroiditis (HT) were studied. Selected patients had active disease and high levels of serum anti-dsDNA antibodies (IgG, IgM and IgA) or of serum anti-TPO (thyroid peroxidase) antibodies and of anti-Tg (thyroglobulin) antibodies (IgG). Serum was collected from patients by peripheral venepuncture.

**Healthy donors' sera**

Healthy individuals with no history of SLE or of HT were studied. Human serum from healthy donors was collected by peripheral venepuncture. Serum was pooled from 10 to 20 donors. For each experiment, the pooled serum contained an equal volume of serum from each donor; usually 3 ml from each donor were used. Prior to chromatography serum was heat inactivated at 56°C for 30 min and then filtered through a 0.2 $\mu$m-pore size Millipore filter.

**Preparation of GammaBind G Sepharose flow-through fraction (GBF fraction)**

2 ml of normal human serum (NHS), i.e. 18.0 mg of IgG, was dialysed by centrifugation (MW limit 50K Da) in a binding buffer, and then was loaded into a 2.0 ml GammaBind G Sepharose column. The column capacity was 18 mg of human IgG per 1 ml of drained gel. The GBF fraction thus obtained was dialysed and concentrated by centrifugation in PBS before being used for measurement of Ig class concentration by capture ELISA. GBF fractions with IgG concentrations of at least 10 μg per ml were used for the separation of IgG-reactive Abs.

**Affinity chromatography and capture ELISA**

Separation of IgG-reactive antibodies from total NHS IgG (affinity purified on GammaBind G Sepharose), and from the GBF (GammaBind G Flowthrough) fraction was performed on IgG-Sepharose (IgG-coupled to CNBr-activated Sepharose 4B), as presented in the above disclosure of the invention. Samples of affinity purified IgG-reactive Abs obtained from six independent runs were concentrated by ultrafiltration (YM-50, cut-off MW 50,000). Ig concentration was adjusted to 100 μg per ml for the IgG-reactive antibody preparations obtained both from total NHS IgG and from the GBF fraction. Obtained IgG-reactive antibodies were tested for the presence of $IgG_2$ monomers and dimers, RF (Rheumatoid Factor) activity and for the ability to neutralize autoantibodies present in the serum of SLE and of HT patients.

**Detection of autoantibodies by indirect solid phase enzyme immunoassay (ELISA)**

The quantitative measurement of autoantibodies reactive with the antibody Fc portion (as a measure of RF activity), with dsDNA, with Tg and with TPO was carried out using the following ELISA kits, produced by ORGENTEC Diagnostika GmbH.

*1. ORG 522S Rheumatoid factor screen*

Immunometric Enzyme Immunoassay for the quantitative measurement of IgG, IgM and IgA rheumatoid factor in serum or plasma (cut off 25 U/ml)

*2. ORG 604S Anti-dsDNA Ab screen*

Immunometric Enzyme Immunoassay for the quantitative measurement of IgG, IgM and IgA autoantibodies to double-stranded (ds) DNA (cut-off 25 U/ml)

*3. ORG 503 Anti-TPO Ab screen*

Immunometric Enzyme Immunoassay for the quantitative measurement of IgG antibodies against TPO (borderline 50-75 IU/ml)

*4. ORG 503 Anti-Tg Ab screen*

Immunometric Enzyme Immunoassay for the quantitative measurement of IgG antibodies against Tg (borderline 100-150 IU/ml).

**Neutralization of autoantibodies by IgG-reactive antibodies**

Inhibition of the binding capacity of each autoantibody was calculated as follows:

$$\frac{\text{autoantibodies binding in the presence of IgG-reactive antibodies}}{\text{autoantibodies binding in the absence of IgG-reactive antibodies}} \times 100$$

The concentration of 50μg per ml of IgG-reactive antibodies used in the neutralization assay corresponds to about 50μg of IgG per ml of 1:200 diluted patient's serum (assuming that human serum has 10mg of IgG per ml).

Determination of rheumatoid factor (RF) activity

The RF activity present in IgG-reactive antibody preparations was determined using Ab concentration of 50μg per ml.

**Detection of anti-dsDNA antibodies and their neutralization with IgG-reactive Abs**

Anti-dsDNA activity in patients' sera was detected by an ELISA screening kit. Values greater than 25 U/ml of anti-dsDNA Abs at serum dilutions 1:100 were regarded as positive (as recommended by the manufacturer).

Whenever the level of the anti-dsDNA Abs at 1:100 dilution exceeded the highest value on the standard curve, we used serum dilutions with anti-dsDNA Ab concentrations fitting the linear portion of the standard curve. In this way, the neutralization activity of IgG-reactive Abs was assayed by co-incubation of patient's serum, at selected dilution(s) with anti-dsDNA Ab level expressed in U/ml within the linear standard curve range, usually with 50 $\mu$g/ml of IgG-reactive Abs. Dose-dependent inhibition of binding was assayed at concentrations of 50$\mu$g and of 200 $\mu$g per ml of IgG-reactive Abs obtained from un-fractionated total NHS IgG, as well as at concentrations of 50 $\mu$g and of 500 $\mu$g per ml of un-fractionated total NHS IgG.

**Detection of anti-Tg antibodies and anti-TPO antibodies and their neutralization with IgG-reactive Abs**

Anti-Tg and anti-TPO activity in patients' sera were detected by an ELISA screening kit. Values greater than 150 IU/ml for anti-Tg Abs and 75 IU/ml for anti-TPO Abs at serum dilution 1:100 were regarded as positive (as recommended by the manufacturer).

Whenever the level of the anti-Tg Abs and anti-TPO Abs at 1:100 dilution exceeded the highest value of the standard curve, we used serum dilutions with anti-Tg Ab and anti-TPO Ab concentrations fitting the linear portion of the standard curve. In this way, the neutralization activity of IgG-reactive Abs was assayed by co-incubation of patient's serum at selected dilution(s) with anti-Tg Ab and anti-TPO Ab level expressed in IU/ml within the linear standard curve range, usually with 50$\mu$g/ml of IgG-reactive Abs. Dose-dependent inhibition of binding was assayed at concentrations of 50 $\mu$g and of 200 $\mu$g per ml of IgG-reactive Abs obtained from un/fractionated total NHS IgG, as well as at concentrations of 50$\mu$g and of 500 $\mu$g per ml of un/fractionated total NHS IgG.

**SDS-PAGE and Western blotting**

To examine heavy (H) and light chains (L), the IgG-reactive Abs obtained from the GBF fraction and the IgG purified on GammaBind G Sepharose were analyzed by reducing SDS-PAGE on 10% gel. IgG monomers and dimers were analyzed by denaturing but non-reducing SDS-PAGE on 4% Tris-glycine gels as described previously (Yoo EM et al., 2003). The SDS-PAGE-resolved antibodies were electrophoretically transferred to a nitrocellulose membrane. Nonspecific sites were blocked by incubating the membrane for 1 h at room temperature in PBS containing 0.2% (v/v) Tween 20 and 5% (w/v) nonfat dried milk. For immunodetection, mouse mAbs specific for human $\gamma$1 and $\gamma$2 were used as a primary reagent for overnight incubation at 4°C. After washing, the blot was probed with peroxidase-conjugated goat anti-mouse IgG for 1 h at room temperature. Detection was performed using the Supersignal Substrate system (Pierce) (as recommended by the manufacturer).

**REFERENCES**

1. Davidson A, Diamond B. Autoimmune diseases. N Engl J Med. 2001;345:340-50.

2. Murakami M, Honjo T. Transgenic mouse models for B-cell dominant autoimmune diseases. Curr Opin Immunol. 1997;9:846-50.

3. Shoenfeld Y. Common infections, idiotypic dysregulation, autoantibody spread and induction of autoimmune diseases. J Autoimmun. 1996;9:235-9.

4. Bockenstedt LK, Gee RJ, Mamula MJ. Self-peptides in the initiation of lupus autoimmunity. J Immunol. 1995;154: 3516-24.

5. Cancro MP, Kearney JF. B cell positive selection: road map to the primary repertoire? J Immunol. 2004;173:15-9.

6. Boyden SV. Natural antibodies and the immune response. Adv Immunol. 1966;5:1-28.

7. Rodman TC, To SE, Sullivan JJ, Winston R. Innate natural antibodies. Primary roles indicated by specific epitopes. Hum Immunol. 1997;55:87-95.

8. Casali P, Schettino EW. Structure and function of natural antibodies. Curr Top Microbiol Immunol. 1996;210: 167-79.

9. Dighiero G, Rose NR. Critical self-epitopes are key to the understanding of self-tolerance and autoimmunity. Immunol Today. 1999; 20:423-8.

10. Silverstein AM, Rose NR. There is only one immune system! The view from immunopathology. Semin Immunol. 2000;12: 173-8; discussion 257-344.

11. Martin F, Oliver AM, Kearney JF. Marginal zone and B1 B cells unite in the early response against T-independent blood-borne particulate antigens.Immunity. 2001;14: 617-629.

12. Kreutzmann S, Manuela M, Weber H, et al. Human immunoglobulin M memory Bcells controlling Streptococcus pneumoniae infections are generated in the spleen. J Exp Med. 2003;197:939-945.

13. Melero J, Tarragó D, Nuñez-Roldán A, Sánchez B. Human polyreactive IgM monoclonal antibodies with blocking activity against self-reactive IgG. Scand J Immunol. 1997 Apr; 45(4):393-400.

14. Rossi F, Dietrich G, Kazatchkine MD. Anti-idiotypes against autoantibodies in normal immunoglobulins: evidence for network regulation of human autoimmune responses. Immunol Rev. 1989; 110:135-49.

15. Imbach P, Barandun S, d'Apuzzo V, Baumgartner C, Hirt A, Morell A, Rossi E, Schöni M, Vest M, Wagner HP. High-dose intravenous gammaglobulin for idiopathic thrombocytopenic purpura in childhood. Lancet. 1981;1: 1228-31.

16. Dwyer JM. Manipulating the immune system with immune globulin. N Engl J Med. 1992; 326:107-16.

17. Kohler H. Superantibodies: synergy of innate and acquired immunity. Appl Biochem Biotechnol.2000 Jan-Mar; 83(1-3):1-9; discussion 10-2, 145-53.

18. Fuchs S, Feferman T, Meidler R, Brenner T, Laub O, Souroujon MC. The disease-specific arm of the therapeutic effect of intravenous immunoglobulin in autoimmune diseases: Experimental Autoimmune Myasthenia Gravis as a Model. IMAJ 2008;10:58-60.

19. Stewart AK, Huang C, Long AA, Stollar BD, Schwartz RS. VH-gene representation in autoantibodies reflects the normal human B-cell repertoire. Immunol Rev. 1992; 128:101-22.

20. Kohsaka H, Carson DA, Rassenti LZ, Ollier WE, Chen PP, Kipps TJ, Miyasaka N. The human immunoglobulin V(H) gene repertoire is genetically controlled and unaltered by chronic autoimmune stimulation. J Clin Invest. 1996; 98:2794-800.

21. Foreman AL, Van de Water J, Gougeon ML, Gershwin ME. B cells in autoimmune diseases: insights from analyses of immunoglobulin variable(Ig V) gene usage. Autoimmun Rev. 2007;6:387-401. Epub 2007 Jan 12.

22. Hahn BH. Antibodies to DNA. N Engl J Med 1998;338(19):1359-68.

23. Swaak T, Smeenk R. Detection of anti-dsDNA as a diagnostic tool: a prospective study in 441 non systemic lupus erythematosus patients with anti-dsDNA antibody (anti-dsDNA). Ann Rheum Dis 1985; 44(4):245-51

24. Munoz LE, Gaipl US, Herrmann M. Predictive value of anti-dsDNA autoantibodies: Importance of the assay. Autoimmunity Reviews 2008; 7:594-597.

25. Blank M, Shoenfeld Y. The story of the 16/6 idiotype and systemic lupus erythematosus. Isr Med Assoc J. 2008; 10:37-9.

26. Madaio MP, Hodder S, Schwartz RS, Stollar BD. Responsiveness of autoimmune and normal mice to nucleic acid antigens. J Immunol. 1984;132:872-6.

27. Li Y, Spellerberg MB, Stevenson FK, Capra JD, Potter KN. The binding specificity of human VH 4-34 (VH 4-21) encoded antibodies is determined by both VH framework region 1 and complementarity determining region 3. J Mol Biol. 1996 Mar 1;256(3):577-89.

28. Spellerberg MB, Chapman CJ, Mockridge CI, Isenberg DA, Stevenson FK. Dual recognition of lipid A and DNA by human antibodies encoded by the VH4-21 gene: a possible link between infection and lupus. Hum Antibodies Hybridomas. 1995;6:52-6.

29. Singh RR, Hahn BH, Tsao BP, Ebling FM. Evidence for multiple mechanisms of polyclonal T cell activation in murine lupus. J Clin Invest. 199815;102:1841-9.

30. Singh RR, Ebling FM, Sercarz EE, Hahn BH. Immune tolerance to autoantibody-derived peptides delays development of autoimmunity in murine lupus. J Clin Invest. 1995;96:2990-6.

31. Ebling FM, Tsao BP, Singh RR, Sercarz E, Hahn BH. A peptide derived from an autoantibody can stimulate T cells in the (NZB x NZW)F1 mouse model of systemic lupus erythematosus. Arthritis Rheum. 1993;36:355-64.

32. Demaison C, David D, Fautrel B, Theze J. V(H) gene-family representation in peripheral activated B cells from systemic lupus erythematosus (SLE) patients. Clin Exp Immunol. 1996 Jun;104(3):439-45.

33. Singh AK, Yang JQ, Parekh VV, Wei J, Wang CR, Joyce S, Singh RR, Van Kaer L. The natural killer T cell ligand alpha-galactosylceramide prevents or promotes pristane-induced lupus in mice. Eur J Immunol. 2005;35: 1143-54.

34. Mendlovic S, Brocke S, Shoenfeld Y, Ben-Bassat M, Meshorer A, Bakimer R, Mozes E. Induction of a systemic lupus erythematosus-like disease in mice by a common human anti-DNA idiotype. Proc Natl Acad Sci U S A. 1988; 85:2260-4.

35. Eivazova ER, McDonnell JM, Sutton BJ, Staines NA. Cross-reactivity of anti-idiotypic antibodies with DNA in systemic lupus erythematosus. Arthritis Rheum. 2000;43:429-39.

36. Wun HL, Leung DT, Wong KC, Chui YL, Lim PL. Molecular mimicry: anti-DNA antibodies may arise inadvertently as a response to antibodies generated to microorganisms. Int Immunol. 2001 Sep;13(9):1099-107.

37. Sthoeger ZM, Sharabi A, Dayan M, Zinger H, Asher I, Sela U, Mozes E. The tolerogenic peptide hCDR1 downregulates pathogenic cytokines and apoptosis and upregulates immunosuppressive molecules and regulatory T cells in peripheral blood mononuclear cells of lupus patients. Hum Immunol. 2009;70:139-45.

38. Toubi E, Kessel A, Shoenfeld Y. High-dose intravenous immunoglobulins: an option in the treatment of systemic lupus erythemathosus. Human Immunology 2005; 66:395-402.

39. Ahsan N. Intravenous immunoglobulin induced-nephropathy: a complication of IVIg therapy. J Nephrol 1998; 11:175.

40. Shoenfeld Y, Rauova L, Gilburd B, Kvapil F, Goldberg I, Kopolovic J, Rovensky J, Blank M. Efficacy of IVIG affinity-purified anti-double-stranded DNA anti-idiotypic antibodies in the treatment of an experimental murine model of systemic lupus erythemathosus. International Immunology 2002; 14:1303-1311.

41. McLachlan SM, Rapoport B. Why measure thyroglobulin autoantibodies rather than thyroid peroxidase autoantibodies?. Thyroid. 2004; 14:510-20.

42. Chardès T, Chapal N, Bresson D, Bès C, Giudicelli V, Lefranc MP, Péraldi-Roux S. The human anti-thyroid peroxidase autoantibody repertoire in Graves' and Hashimoto's autoimmune thyroid diseases. Immunogenetics. 2002;54:141-57.

43. Prentice L, Kiso Y, Fukuma N, Horimoto M, Petersen V, Grennan F, Pegg C, Furmaniak J, Rees Smith B. Monoclonal thyroglobulin autoantibodies: variable region analysis and epitope recognition. J Clin Endocrinol Metab. 1995;80:977-86.

44. Bresson D, Rebuffat SA, Péraldi-Roux S. Localization of the immunodominant region on human thyroid peroxidase in autoimmune thyroid diseases: an update. J Autoimmune Dis. 2005;2:2.

45. Canton A, de Fabregas O, Tintore M, et al. Encephalopathy associated to autoimmune thyroid disease: a more appropriate term for an underestimated condition? J Neurol Sci 2000;176:65-69.

46. Dietrich G, Kazatchkine MD. Normal immunoglobulin G (IgG) for therapeutic use (intravenous Ig) contain anti-idiotypic specificities against an immunodominant, disease-associated, cross-reactive idiotype of human anti-thyroglobulin autoantibodies. J Clin Invest. 1990;85:620-5.

47. Beardsley DS. ITP in the 21 st century. Hematology Am Soc Hematol Educ Program. 2006:402-7.

48. Stahl D, Hoemberg M, Cassens U, Pachmann U, Sibrowski W. Evidence that human autoimmune thrombocytopenia mediated by both immunoglobulin isotypes IgM and IgG is an independent disease entity. Eur J Haematol. 2005;75:318-27.

49. Chang M, Nakagawa PA, Williams SA, Schwartz MR, Imfeld KL, Buzby JS, Nugent DJ. Immune thrombocytopenic purpura (ITP) plasma and purified ITP monoclonal autoantibodies inhibit megakaryocytopoiesis in vitro. Blood. 2003 Aug 1;102(3):887-95. Epub 2003 Apr 3.

50. Lipp E, von Felten A, Sax H, Müller D, Berchtold P. Antibodies against platelet glycoproteins and antiphospholipid antibodies in autoimmune thrombocytopenia. Eur J Haematol. 1998;60:283-8.

51. McMillan R. The pathogenesis of chronic immune (idiopathic) thrombocytopenic purpura. Semin Hematol. 2000; 37(1 Suppl 1):5-9.

52. Hou M, Stockelberg D, Kutti J, Wadenvik H. Antibodies against platelet GPIb/IX, GPIIb/IIIa, and other platelet antigens in chronic idiopathic thrombocytopenic purpura. Eur J Haematol. 1995 Nov;55(5):307-14.

53. Crow AR, Lazarus AH. Role of Fcgamma receptors in the pathogenesis and treatment of idiopathic thrombocytopenic purpura. J Pediatr Hematol Oncol. 2003;25 Supp1 1:S14-8.

54. Bussel JB. Fc receptor blockade and immune thrombocytopenic purpura. Semin Hematol. 2000;37:261-6.

55. Crow AR, Song S, Freedman J, Helgason CD, Humphries RK, Siminovitch KA, Lazarus AH. IVIg-mediated amelioration of murine ITP via FcgammaRIIB is independent of SHIP1, SHP-1, and Btk activity. Blood. 2003;102: 558-60.

56. Siragam V, Brinc D, Crow AR, Song S, Freedman J, Lazarus AH. Can antibodies with specificity for soluble antigens mimic the therapeutic effects of intravenous IgG in the treatment of autoimmune disease?. J Clin Invest. 2005;115:155-60.

57. Denomme GA, Mahmoudi M, Cairns E, Bell DA. Immunoglobulin V region sequences of two human antiplatelet monoclonal autoantibodies derived from B cells of normal origin. J Autoimmun. 1994;7:521-35.

58. Roark JH, Bussel JB, Cines DB, Siegel DL. Genetic analysis of autoantibodies in idiopathic thrombocytopenic purpura reveals evidence of clonal expansion and somatic mutation. Blood. 2002;100:1388-98.

59. Carson DA, Chen PP, Fox RI, Kipps TJ, Jirik F, Goldfien RD, Silverman G, Radoux V, Fong S. Rheumatoid factor and immune networks. Annu Rev Immunol. 1987; 5: 109-26.

60. Boruchov AM, Heller G, Veri MC, Bonvini E, Ravetch JV, Young JW. Activating and inhibitory IgG Fc receptors on human DCs mediate opposing functions. J Clin Invest. 2005; 115: 2914-23.

61. Liu Y, Gao X, Masuda E, Redecha PB, Blank MC, Pricop L. Regulated expression of FcgammaR in human dendritic cells controls cross-presentation of antigen-antibody complexes. J Immunol. 2006 Dec 15;177(12):8440-7.

62. Sharabi A, Mozes E. The suppression of murine lupus by a tolerogenic peptide involves foxp3-expressing CD8 cells that are required for the optimal induction and function of foxp3-expressing CD4 cells. J Immunol. 2008;181:

3243-51.

63. Kazatchkine MD, Kaveri SV. Advances in immunology: immunomodulation of autoimmune and inflammatory diseases with intravenous immune globulin. N Engl J Med 2001; 345:747-55.

64. Dalakas MC. The use of intravenous immunoglobulin in the treatment of autoimmune neuromuscular diseases: evidence-based indications and safety profile. Pharmacology & Therapeutics 2004;102:177-193.

65. EFNS guidelines for the use of intravenous immunoglobulin in treatment of neurological diseases. European J Neurology 2008; 15:893-908.

66. Solomon B. Intravenous immunoglobulin and Alzheimer's disease immunotherapy. Current Opinion in Molecular Therapeutics 2007;9:79-85.

67. Mouthon L, Kaveri SV, Spalter SH, Lacroix-Desmazes S, Lefranc C, Desai R, Kazatchkine MD. Mechanism of action of intravenous immunoglobulin therapy in immune-mediated diseases. Clin Exp Immunol, 1996; 104 (suppl. 1):3-9.

68. Seite JF, Shoenfeld Y, Youinou P, Hillion S. What is the content of the magic draft IVIg? Autoimmunity Reviews 2008;7: 435-439.

69. Yoo EM, Wims LA, Chan LA, Morrison SL. Human IgG2 can form covalent dimers?. J Immunol 2003; 170: 3134-3138).

## Claims

1. An immunoglobulin preparation containing natural polyclonal IgG-reactive antibodies (Abs) isolated from normal human serum (NHS), for the treatment of autoimmune diseases and immune system disorders.

2. The preparation of claim 1, wherein the IgG-reactive Abs bind pathologic autoreactive Abs present in the serum of patients with autoimmune diseases and immune system disorders, neutralizing their interaction with self antigens.

3. The preparation of claim 1, wherein the IgG-reactive Abs reduce the binding of anti-dsDNA autoantibodies from Systemic Lupus Erythematosus (SLE) sera to dsDNA immobilized on plates; and of anti-thyroglobulin (Tg) and anti-thyroid peroxidase (TPO) autoantibodies from Hashimoto's thyroiditis (HT) sera to Tg and TPO, respectively.

4. The preparation of claim 1, wherein the IgG-reactive Abs are able to interact with B cell receptors, Fc receptors, MHC complex, T regulatory cell receptors as well as with DC surface FcγRIIa.

5. The preparation of claims 1-4, wherein the IgG-reactive antibodies are obtained by:

   (a) passing NHS through a chromatographic matrix that specifically binds human IgG, preferably GammaBind G Sepharose matrix and eluting the matrix-bound IgG fraction to obtain affinity purified IgG;
   (b) covalent binding of one aliquot of the affinity purified IgG obtained in (a), to CNBr-activated Sepharose 4B, or an equivalent matrix, to obtain IgG-Sepharose;
   (c) contacting a second aliquot of the affinity purified IgG obtained in (a) with the IgG-Sepharose obtained in (b) and eluting IgG-Sepharose bound Igs to prepare IgG-reactive IgG.

6. The preparation of claims 1-4, wherein the IgG-reactive antibodies are obtained by:

   (a') passing NHS through a chromatographic matrix that specifically binds human IgG, preferably through GammaBind G Sepharose matrix and recovering the matrix-bound IgG fraction and the matrix-unbound fraction or "GammaBind G flow-through" (GBF) fraction;
   (b') eluting the matrix-bound IgG fraction, obtained as in (a'), to obtain affinity-purified IgG;
   (c') covalent binding of the affinity-purified IgG obtained in (b') to CNBr-activated Sepharose 4B, or an equivalent matrix, to prepare IgG-Sepharose;
   (d') contacting the GBF fraction obtained in (a') with IgG-Sepharose and eluting the IgG-Sepharose bound Igs to obtain IgG-reactive (GBF) Abs.

7. The preparation of claims 1-4, containing a combination of IgG-reactive IgG and IgG-reactive GBF Abs according to claims 5 and 6, respectively.

8. The preparation of claims 1-7, wherein the IgG-reactive Abs are enriched in IgG$_2$.

9. The preparation of claim 8, wherein said IgG$_2$ are IgG$_2$ dimers.

10. The preparation of any preceding claims, for use in the treatment of Systemic lupus erythematosus (SLE) and Hashimoto's thyroiditis.

11. The preparation of any preceding claims, for use in the treatment of:

   - hematologic autoimmune disease including immune thrombocytopenic purpura, autoimmune haemolytic anemia, acquired hemophilia, Evans syndrome;
   - autoimmune inflammatory myopathies including polymyositis and dermatomyositis;
   - rheumatologic disease including rheumatoid arthritis, antiphospholipid antibody syndrome, systemic vasculitides and anti-neutrophil cytoplasmic autoantibody disorders;
   - autoimmune diseases associated with AIDS;
   - organ-specific autoimmune diseases including autoimmune diabetes mellitus, autoimmune Graves ophthalmopathy, autoimmune uveitis, autoimmune liver disease;
   - autoimmune neurologic disorders including peripheral neuropathies, in particular Ouillain-Barré syndrome, chronic inflammatory demyelinating polyneuropathy, multifocal motor neuropathy, IgM antimyelin-associated glycoprotein paraprotein-associated peripheral neuropathy; neuromuscular junction syndromes, myasthenia gravis, Lambert-Eaton myasthenic syndrome; multiple sclerosis, Lennox-Gastaut syndrome, Rasmussen syndrome;
   - Mixed connective tissue disease;
   - Kawasaki disease;
   - autoimmune dermatologic disorders including toxic epidermal necrolysis and Stevens-Johnson syndrome, autoimmune blistering diseases, atopic dermatitis, chronic urticaria, pemphigus;
   - autoimmune neurocognitive disorders, including pediatric autoimmune neuropsychiatric disorders associated with streptococcal infection.
   - Graft-versus-host disease.

12. A pharmaceutical composition for the treatment of autoimmune diseases or immune system disorders comprising an effective amount of the preparation of claims 1-9.

13. A pharmaceutical composition according to claim 12, to be administered via subcutaneous, intramuscular or intravenous route.

14. A pharmaceutical composition according to claim 12, to be administered as add-on therapy and steroid-sparing agent in patients suffering from autoimmune diseases and immune system disorders.

15. A pharmaceutical composition according to claim 12, containing recombinant monoclonal or polyclonal IgG-reactive IgG$_2$.

**Figure 1**

**Figure 2A**

**Figure 2B**

**Figure 3**

Figure 4

**Figure 5A**

**Figure 5B**

**Figure 6**

**Figure 7**

**Figure 8A**

**Figure 8 B**

**Figure 9**

**Figure 10**

A

B

**Figure 11**

**Figure 12**

Europäisches
Patentamt
European
Patent Office
Office européen
des brevets

## EUROPEAN SEARCH REPORT

Application Number

EP 09 16 1247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEMIEUX REAL ET AL: "Autoantibody-induced formation of immune complexes in normal human serum" CURRENT PHARMACEUTICAL DESIGN, vol. 12, no. 2, 2006, pages 173-179, XP009119385 ISSN: 1381-6128 The whole document, in particular * page 175, column 1, paragraph 2 - column 2, paragraph 1 * | 1-4 | INV. C07K16/42 C07K1/22 A61K39/395 |
| X | SHOENFELD Y ET AL: "EFFICACY OF IVIG AFFINITY-PURIFIED ANTI-DOUBLE-STRANDED DNA ANTI-IDIOTYPIC ANTIBODIES IN THE TREATMENT OF AN EXPERIMENTAL MURINE MODEL OF SYSTEMIC LUPUS ERYTHEMATOSUS" INTERNATIONAL IMMUNOLOGY, OXFORD UNIVERSITY PRESS, GB, vol. 14, no. 11, 1 November 2002 (2002-11-01), pages 1303-1311, XP009012195 ISSN: 0953-8178 * the whole document * | 1-4,10, 11 | |
| X | DIETRICH G ET AL: "A V REGION-CONNECTED AUTOREATIVE SUBFRACTION OF NORMAL HUMAN SERUM IMMUNOGLOBULIN G*" EUROPEAN JOURNAL OF IMMUNOLOGY, WILEY - V C H VERLAG GMBH & CO. KGAA, DE, vol. 22, no. 7, 1 July 1992 (1992-07-01), pages 1701-1706, XP000877158 ISSN: 0014-2980 The whole document, in particular * page 1705, paragraph 3 * | 1-9, 12-15 | |

TECHNICAL FIELDS
SEARCHED      (IPC)

C07K

-/--

The present search report has been drawn up for all claims

| Place of search | Date of completion of the search | Examiner |
|---|---|---|
| Munich | 6 July 2009 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 09 16 1247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRULEY-ROSSET MARTINE ET AL: "Polyreactive autoantibodies purified from human intravenous immunoglobulins prevent the development of experimental autoimmune diseases." LABORATORY INVESTIGATION, vol. 83, no. 7, July 2003 (2003-07), pages 1013-1023, XP002535282 ISSN: 0023-6837 * the whole document * | 1-9, 12-15 | |
| X | VASSILEV T L ET AL: "Variable Region-Connected, Dimeric Fraction of Intravenous immunoglobulin Enriched in Natural Autoantibodies" JOURNAL OF AUTOIMMUNITY, LONDON, GB, vol. 8, no. 3, 1 January 1995 (1995-01-01), pages 405-413, XP002265834 ISSN: 0896-8411 The whole document, in particular * page 406, paragraph 2 - page 407, paragraph 1 * * page 412, line 10 - line 16 * | 1-9, 12-15 | |
| X | DWYER J M: "MANIPULATING THE IMMUNE SYSTEM WITH IMMUNE GLOBULIN" NEW ENGLAND JOURNAL OF MEDICINE, THE, MASSACHUSETTS MEDICAL SOCIETY, WALTHAM, MA, US, vol. 326, no. 2, 9 January 1992 (1992-01-09), pages 107-116, XP001119804 ISSN: 0028-4793 The whole document, in particular * tables 1-3 * | 1-4,10, 11 | TECHNICAL FIELDS SEARCHED (IPC) |

-/--

The present search report has been drawn up for all claims

| Place of search | Date of completion of the search | Examiner |
|---|---|---|
| Munich | 6 July 2009 | Chapman, Rob |

EPO FORM 1503 03.82 (P04C01)

Europäisches
Patentamt
European
Patent Office
Office européen
des brevets

## EUROPEAN SEARCH REPORT

Application Number

EP 09 16 1247

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 394 183 A (HEMA QUEBEC [CA]) 3 March 2004 (2004-03-03) * the whole document * ----- | | |

TECHNICAL FIELDS
SEARCHED      (IPC)

The present search report has been drawn up for all claims

| Place of search | Date of completion of the search | Examiner |
|---|---|---|
| Munich | 6 July 2009 | Chapman, Rob |

EPO FORM 1503 03.82 (P04C01)

## ANNEX TO THE EUROPEAN SEARCH REPORT
## ON EUROPEAN PATENT APPLICATION NO.

EP 09 16 1247

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2009

| Patent document cited in search report | | Publication date | Patent family member(s) | Publication date |
|---|---|---|---|---|
| EP 1394183 | A | 03-03-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03099868 A
- US 6932969 B, Bourel D

### Non-patent literature cited in the description

- **Davidson A ; Diamond B.** Autoimmune diseases. *N Engl J Med.,* 2001, vol. 345, 340-50
- **Murakami M ; Honjo T.** Transgenic mouse models for B-cell dominant autoimmune diseases. *Curr Opin Immunol.,* 1997, vol. 9, 846-50
- **Shoenfeld Y.** Common infections, idiotypic dysregulation, autoantibody spread and induction of autoimmune diseases. *J Autoimmun.,* 1996, vol. 9, 235-9
- **Bockenstedt LK ; Gee RJ ; Mamula MJ.** Self-peptides in the initiation of lupus autoimmunity. *J Immunol.,* 2004, vol. 154, 3516-24
- **Cancro MP ; Kearney JF.** B cell positive selection: road map to the primary repertoire?. *J Immunol.,* vol. 173, 15-9
- **Boyden SV.** Natural antibodies and the immune response. *Adv Immunol.,* 1966, vol. 5, 1-28
- **Rodman TC ; To SE ; Sullivan JJ ; Winston R.** Innate natural antibodies. Primary roles indicated by specific epitopes. *Hum Immunol.,* 1997, vol. 55, 87-95
- **Casali P ; Schettino EW.** Structure and function of natural antibodies. *Curr Top Microbiol Immunol.,* 1996, vol. 210, 167-79
- **Dighiero G ; Rose NR.** Critical self-epitopes are key to the understanding of self-tolerance and autoimmunity. *Immunol Today,* 1989, vol. 20, 423-8
- **Silverstein AM ; Rose NR.** There is only one immune system! The view from immunopathology. *Semin Immunol.,* 2000, vol. 12, 173-8
- **Martin F ; Oliver AM ; Kearney JF.** Marginal zone and B1 B cells unite in the early response against T-independent blood-borne particulate antigens. *Immunity,* 2001, vol. 14, 617-629
- **Kreutzmann S ; Manuela M ; Weber H et al.** Human immunoglobulin M memory Bcells controlling Streptococcus pneumoniae infections are generated in the spleen. *J Exp Med.,* 2003, vol. 197, 939-945
- **Melero J ; Tarragó D ; Nuñez-Roldán A ; Sánchez B.** Human polyreactive IgM monoclonal antibodies with blocking activity against self-reactive IgG. *Scand J Immunol.,* April 1997, vol. 45 (4), 393-400

- **Rossi F ; Dietrich G ; Kazatchkine MD.** Anti-idiotypes against autoantibodies in normal immunoglobulins: evidence for network regulation of human autoimmune responses. *Immunol Rev.,* 1989, vol. 110, 135-49
- **Imbach P ; Barandun S ; d'Apuzzo V ; Baumgartner C ; Hirt A ; Morell A ; Rossi E ; Schöni M ; Vest M ; Wagner HP.** High-dose intravenous gammaglobulin for idiopathic thrombocytopenic purpura in childhood. *Lancet,* 1981, vol. 1, 1228-31
- **Dwyer JM.** Manipulating the immune system with immune globulin. *N Engl J Med.,* 1992, vol. 326, 107-16
- **Kohler H.** Superantibodies: synergy of innate and acquired immunity. *Appl Biochem Biotechnol.,* January 2000, vol. 83 (1-3), 1-9
- **Fuchs S ; Feferman T ; Meidler R ; Brenner T ; Laub O ; Souroujon MC.** The disease-specific arm of the therapeutic effect of intravenous immunoglobulin in autoimmune diseases: Experimental Autoimmune Myasthenia Gravis as a Model. *IMAJ,* 2008, vol. 10, 58-60
- **Stewart AK ; Huang C ; Long AA ; Stollar BD ; Schwartz RS.** VH-gene representation in autoantibodies reflects the normal human B-cell repertoire. *Immunol Rev.,* 1992, vol. 128, 101-22
- **Kohsaka H ; Carson DA ; Rassenti LZ ; Ollier WE ; Chen PP ; Kipps TJ ; Miyasaka N.** The human immunoglobulin V(H) gene repertoire is genetically controlled and unaltered by chronic autoimmune stimulation. *J Clin Invest.,* 1996, vol. 98, 2794-800
- **Foreman AL ; Van de Water J ; Gougeon ML ; Gershwin ME.** B cells in autoimmune diseases: insights from analyses of immunoglobulin variable(Ig V) gene usage. *Autoimmun Rev.,* 12 January 2007, vol. 6, 387-401
- **Hahn BH.** Antibodies to DNA. *N Engl J Med,* 1998, vol. 338 (19), 1359-68
- **Swaak T ; Smeenk R.** Detection of anti-dsDNA as a diagnostic tool: a prospective study in 441 non systemic lupus erythematosus patients with anti-dsDNA antibody (anti-dsDNA). *Ann Rheum Dis,* 1985, vol. 44 (4), 245-51

- **Munoz LE ; Gaipl US ; Herrmann M.** Predictive value of anti-dsDNA autoantibodies: Importance of the assay. *Autoimmunity Reviews,* 2008, vol. 7, 594-597
- **Blank M ; Shoenfeld Y.** The story of the 16/6 idiotype and systemic lupus erythematosus. *Isr Med Assoc J.,* 2008, vol. 10, 37-9
- **Madaio MP ; Hodder S ; Schwartz RS ; Stollar BD.** Responsiveness of autoimmune and normal mice to nucleic acid antigens. *J Immunol.,* 1984, vol. 132, 872-6
- **Li Y ; Spellerberg MB ; Stevenson FK ; Capra JD ; Potter KN.** The binding specificity of human VH 4-34 (VH 4-21) encoded antibodies is determined by both VH framework region 1 and complementarity determining region 3. *J Mol Biol.,* 01 March 1996, vol. 256 (3), 577-89
- **Spellerberg MB ; Chapman CJ ; Mockridge CI ; Isenberg DA ; Stevenson FK.** Dual recognition of lipid A and DNA by human antibodies encoded by the VH4-21 gene: a possible link between infection and lupus. *Hum Antibodies Hybridomas,* 1995, vol. 6, 52-6
- **Singh RR ; Hahn BH ; Tsao BP ; Ebling FM.** Evidence for multiple mechanisms of polyclonal T cell activation in murine lupus. *J Clin Invest.,* 05 January 1998, vol. 102, 1841-9
- **Singh RR ; Ebling FM ; Sercarz EE ; Hahn BH.** Immune tolerance to autoantibody-derived peptides delays development of autoimmunity in murine lupus. *J Clin Invest.,* 1995, vol. 96, 2990-6
- **Ebling FM ; Tsao BP ; Singh RR ; Sercarz E ; Hahn BH.** A peptide derived from an autoantibody can stimulate T cells in the (NZB x NZW)F1 mouse model of systemic lupus erythematosus. *Arthritis Rheum.,* 1993, vol. 36, 355-64
- **Demaison C ; David D ; Fautrel B ; Theze J.** V(H) gene-family representation in peripheral activated B cells from systemic lupus erythematosus (SLE) patients. *Clin Exp Immunol.,* June 1996, vol. 104 (3), 439-45
- **Singh AK ; Yang JQ ; Parekh VV ; Wei J ; Wang CR ; Joyce S ; Singh RR ; Van Kaer L.** The natural killer T cell ligand alpha-galactosylceramide prevents or promotes pristane-induced lupus in mice. *Eur J Immunol.,* 2005, vol. 35, 1143-54
- **Mendlovic S ; Brocke S ; Shoenfeld Y ; Ben-Bassat M ; Meshorer A ; Bakimer R ; Mozes E.** Induction of a systemic lupus erythematosus-like disease in mice by a common human anti-DNA idiotype. *Proc Natl Acad Sci U S A.,* 1988, vol. 85, 2260-4
- **Eivazova ER ; McDonnell JM ; Sutton BJ ; Staines NA.** Cross-reactivity of anti-idiotypic antibodies with DNA in systemic lupus erythematosus. *Arthritis Rheum.,* 2000, vol. 43, 429-39
- **Wun HL ; Leung DT ; Wong KC ; Chui YL ; Lim PL.** Molecular mimicry: anti-DNA antibodies may arise inadvertently as a response to antibodies generated to microorganisms. *Int Immunol.,* September 2001, vol. 13 (9), 1099-107
- **Sthoeger ZM ; Sharabi A ; Dayan M ; Zinger H ; Asher I ; Sela U ; Mozes E.** The tolerogenic peptide hCDR1 downregulates pathogenic cytokines and apoptosis and upregulates immunosuppressive molecules and regulatory T cells in peripheral blood mononuclear cells of lupus patients. *Hum Immunol.,* 2009, vol. 70, 139-45
- **Toubi E ; Kessel A ; Shoenfeld Y.** High-dose intravenous immunoglobulins: an option in the treatment of systemic lupus erythemathosus. *Human Immunology,* 2005, vol. 66, 395-402
- **Ahsan N.** Intravenous immunoglobulin induced-nephropathy: a complication of IVIg therapy. *J Nephrol,* 1998, vol. 11, 175
- **Shoenfeld Y ; Rauova L ; Gilburd B ; Kvapil F ; Goldberg I ; Kopolovic J ; Rovensky J ; Blank M.** Efficacy of IVIG affinity-purified anti-double-stranded DNA anti-idiotypic antibodies in the treatment of an experimental murine model of systemic lupus erythemathosus. *International Immunology,* 2002, vol. 14, 1303-1311
- **McLachlan SM ; Rapoport B.** Why measure thyroglobulin autoantibodies rather than thyroid peroxidase autoantibodies?. *Thyroid,* vol. 14, 510-20
- **Chardès T ; Chapal N ; Bresson D ; Bès C ; Giudicelli V ; Lefranc MP ; Péraldi-Roux S.** The human anti-thyroid peroxidase autoantibody repertoire in Graves' and Hashimoto's autoimmune thyroid diseases. *Immunogenetics,* 2002, vol. 54, 141-57
- **Prentice L ; Kiso Y ; Fukuma N ; Horimoto M ; Petersen V ; Grennan F ; Pegg C ; Furmaniak J ; Rees Smith B.** Monoclonal thyroglobulin autoantibodies: variable region analysis and epitope recognition. *J Clin Endocrinol Metab.,* 1995, vol. 80, 977-86
- **Bresson D ; Rebuffat SA ; Péraldi-Roux S.** Localization of the immunodominant region on human thyroid peroxidase in autoimmune thyroid diseases: an update. *J Autoimmune Dis.,* 2005, vol. 2, 2
- **Canton A ; de Fabregas O ; Tintore M et al.** Encephalopathy associated to autoimmune thyroid disease: a more appropriate term for an underestimated condition?. *J Neurol Sci,* vol. 176, 65-69
- **Dietrich G ; Kazatchkine MD.** Normal immunoglobulin G (IgG) for therapeutic use (intravenous Ig) contain antiidiotypic specificities against an immunodominant, disease-associated, cross-reactive idiotype of human anti-thyroglobulin autoantibodies. *J Clin Invest.,* 1990, vol. 85, 620-5
- **Beardsley DS.** ITP in the 21 st century. *Hematology Am Soc Hematol Educ Program.,* 2006, 402-7

- **Stahl D ; Hoemberg M ; Cassens U ; Pachmann U ; Sibrowski W.** Evidence that human autoimmune thrombocytopenia mediated by both immunoglobulin isotypes IgM and IgG is an independent disease entity. *Eur J Haematol.,* 2005, vol. 75, 318-27
- **Chang M ; Nakagawa PA ; Williams SA ; Schwartz MR ; Imfeld KL ; Buzby JS ; Nugent DJ.** Immune thrombocytopenic purpura (ITP) plasma and purified ITP monoclonal autoantibodies inhibit megakaryocytopoiesis in vitro. *Blood,* 03 April 2003, vol. 102 (3), 887-95
- **Lipp E ; von Felten A ; Sax H ; Müller D ; Berchtold P.** Antibodies against platelet glycoproteins and antiphospholipid antibodies in autoimmune thrombocytopenia. *Eur J Haematol.,* 1998, vol. 60, 283-8
- **McMillan R.** The pathogenesis of chronic immune (idiopathic) thrombocytopenic purpura. *Semin Hematol.,* 2000, vol. 37 (1), 5-9
- **Hou M ; Stockelberg D ; Kutti J ; Wadenvik H.** Antibodies against platelet GPIb/IX, GPIIb/IIIa, and other platelet antigens in chronic idiopathic thrombocytopenic purpura. *Eur J Haematol.,* November 1995, vol. 55 (5), 307-14
- **Crow AR ; Lazarus AH.** Role of Fcgamma receptors in the pathogenesis and treatment of idiopathic thrombocytopenic purpura. *J Pediatr Hematol Oncol.,* 2003, vol. 25 (1), 14-8
- **Bussel JB.** Fc receptor blockade and immune thrombocytopenic purpura. *Semin Hematol.,* 2000, vol. 37, 261-6
- **Crow AR ; Song S ; Freedman J ; Helgason CD ; Humphries RK ; Siminovitch KA ; Lazarus AH.** IVIg-mediated amelioration of murine ITP via FcgammaRIIB is independent of SHIP1, SHP-1, and Btk activity. *Blood,* 2003, vol. 102, 558-60
- **Siragam V ; Brinc D ; Crow AR ; Song S ; Freedman J ; Lazarus AH.** Can antibodies with specificity for soluble antigens mimic the therapeutic effects of intravenous IgG in the treatment of autoimmune disease?. *J Clin Invest.,* vol. 115, 155-60
- **Denomme GA ; Mahmoudi M ; Cairns E ; Bell DA.** Immunoglobulin V region sequences of two human antiplatelet monoclonal autoantibodies derived from B cells of normal origin. *J Autoimmun.,* 1994, vol. 7, 521-35
- **Roark JH ; Bussel JB ; Cines DB ; Siegel DL.** Genetic analysis of autoantibodies in idiopathic thrombocytopenic purpura reveals evidence of clonal expansion and somatic mutation. *Blood,* 2002, vol. 100, 1388-98
- **Carson DA ; Chen PP ; Fox RI ; Kipps TJ ; Jirik F ; Goldfien RD ; Silverman G ; Radoux V ; Fong S.** Rheumatoid factor and immune networks. *Annu Rev Immunol.,* 1987, vol. 5, 109-26
- **Boruchov AM ; Heller G ; Veri MC ; Bonvini E ; Ravetch JV ; Young JW.** Activating and inhibitory IgG Fc receptors on human DCs mediate opposing functions. *J Clin Invest.,* 2005, vol. 115, 2914-23
- **Liu Y ; Gao X ; Masuda E ; Redecha PB ; Blank MC ; Pricop L.** Regulated expression of FcgammaR in human dendritic cells controls cross-presentation of antigen-antibody complexes. *J Immunol.,* 15 December 2006, vol. 177 (12), 8440-7
- **Sharabi A ; Mozes E.** The suppression of murine lupus by a tolerogenic peptide involves foxp3-expressing CD8 cells that are required for the optimal induction and function of foxp3-expressing CD4 cells. *J Immunol.,* 2008, vol. 181, 3243-51
- **Kazatchkine MD ; Kaveri SV.** Advances in immunology: immunomodulation of autoimmune and inflammatory diseases with intravenous immune globulin. *N Engl J Med,* 2001, vol. 345, 747-55
- **Dalakas MC.** The use of intravenous immunoglobulin in the treatment of autoimmune neuromuscular diseases: evidence-based indications and safety profile. *Pharmacology & Therapeutics,* 2004, vol. 102, 177-193
- EFNS guidelines for the use of intravenous immunoglobulin in treatment of neurological diseases. *European J Neurology,* 2008, vol. 15, 893-908
- **Solomon B.** Intravenous immunoglobulin and Alzheimer's disease immunotherapy. *Current Opinion in Molecular Therapeutics,* 2007, vol. 9, 79-85
- **Mouthon L ; Kaveri SV ; Spalter SH ; Lacroix-Desmazes S ; Lefranc C ; Desai R ; Kazatchkine MD.** Mechanism of action of intravenous immunoglobulin therapy in immune-mediated diseases. *Clin Exp Immunol,* 1996, vol. 104 (1), 3-9
- **Seite JF ; Shoenfeld Y ; Youinou P ; Hillion S.** What is the content of the magic draft IVIg?. *Autoimmunity Reviews,* vol. 7, 435-439
- **Yoo EM ; Wims LA ; Chan LA ; Morrison SL.** Human IgG2 can form covalent dimers?. *J Immunol,* vol. 170, 3134-3138